# EUROPÄISCHE PATENTANMELDUNG

(11) **EP 1 961 453 A2**
(43) Veröffentlichungstag der Anmeldung: **27.08.2008**
(21) Anmeldenummer: 08158316.3
(22) Anmeldetag: 26.04.2005
(51) Int. Cl.: A61Q 17/04, A61K 8/35, A61K 8/37

(54) **Benzyliden-ß-dicarbonylverbindungen als UV-Absorber**

(30) Priorität: 03.05.2004 DE 102004021805; 17.06.2004 DE 102004029239
(62) Teilanmeldung aus: 05742743.7
(71) Anmelder: Symrise GmbH & Co. KG, 37603 Holzminden (DE)
(72) Erfinder: Koch, Oskar, 37079, Göttingen (DE); Johncock, William, 21465, Reinbek (DE)
(74) Vertreter: Eisenführ, Speiser & Partner

(57) **Zusammenfassung**

Beschrieben wird die Verwendung einer Verbindung der Formel 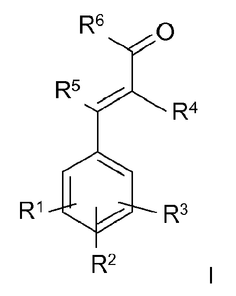 worin
R¹-R³
unabhängig voneinander Wasserstoff, C₁-C₈-Alkyl oder C₁-C₈-Alkoxy,
R⁴
COR, CO₂R, CONR₂ mit R gleich C₁-C₈-Alkyl oder C₃-C₈-Cycloalkyl
R⁵
H oder C₁-C₈-Alkyl,
R⁶
Aryl, mit bis zu drei C₁-C₈-Alkyl- oder C₁-C₈-Alkoxy substituiertes Aryl, oder C₃-C₈-Cycloalkyl
sind,
als UV-Filter in kosmetischen Formulierungen, insbesondere in Kombination mit UV-Filtern aus der Gruppe der Methoxycinnamat-Derivate und/oder Dibenzoylmethan-Derivate, mit der Maßgabe, dass R⁵ gleich H ist, wenn R⁴ gleich COR ist.

## Beschreibung

Die Erfindung betrifft die Verwendung bestimmter Benzyliden-β-dicarbonylverbindungen als UV-Filter (UV-Absorber) in kosmetischen und dermatologischen Formulierungen wie Sonnenschutzmitteln, Tages- und Haarpflegeprodukten. Die Erfindung betrifft ferner entsprechende kosmetische und dermatologische Formulierungen. Schließlich betrifft die Erfindung auch bestimmte neue Benzyliden-β-dicarbonylverbindungen, die als UV-B-Filter eingesetzt werden können.

UV-Strahlen werden nach ihrer Wellenlänge unterteilt in UV-A-Strahlen (320-400nm) und UV-B-Strahlen (280-320nm). Die schädigende Wirkung, das Auftreten von Sonnenbrand (Erythem), wird dabei maßgeblich von der UV-B-Strahlung verursacht. Dermatologische Untersuchungen haben ergeben, daß auch die UV-A-Strahlung Hautschädigungen hervorruft. So wird dieser Bereich der Strahlung für die vorzeitige Hautalterung bis hin zum Hautkrebs verantwortlich gemacht.

Aus dieser Erkenntnis heraus ist es für einen modernen kosmetischen Sonnenschutz unerläßlich, beide Bereiche, sowohl UV-A als auch UV-B abzudecken. Aus diesem Grund sind neben den bereits bekannten UV-B-Absorbern, wie z.B. Campher-Derivate, Salicylsäure-Derivate, Benzophenone, Cinnamate, Benzimidazole sowie Triazine, neue UV-A-Absorber entwickelt worden. Einer der wichtigsten Vertreter ist das 4-Dimethylethyl-4'-methoxydibenzoylmethan.

Nachteilig ist nun, daß bei Einsatz von Kombinationen von UV-A- mit UV-B-Absorbern in Sonnenschutzmitteln teilweise Inkompatibilitäten auftreten. So ist z.B. eine Kombination von 4-Dimethylethyl-4'-methoxydibenzoylmethan und Octylmethoxycinnamat aufgrund von Interaktionen nicht photostabil. Das hat zur Folge, daß die Schutzleistung bei Sonnenbestrahlung rasch abnimmt.

Es war daher die primäre Aufgabe der vorliegenden Erfindung, UV-Filter (UV-Absorber), und zwar insbesondere UV-B-Filter, anzugeben, die nicht nur für sich allein photostabil sind, sondern darüber hinaus bezüglich der Photostabilität kompatible kosmetische Formulierungen mit anderen UV-Filtern bilden. Vorzugsweise sollte dabei die Löslichkeit der UV-Filter in üblichen kosmetischen Ölen hoch sein.

Diese primäre Aufgabe wurde erfindungsgemäß gelöst durch Verwendung der Verbindungen der Formel I 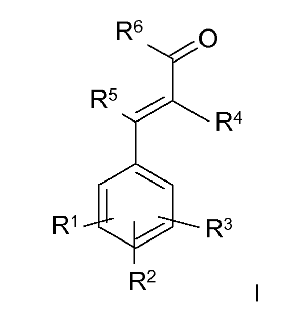 worin
- R¹-R³: unabhängig voneinander Wasserstoff, C₁-C₈-Alkyl oder C₁-C₈-Alkoxy,
- R⁴: COR, CO₂R, CONR₂ mit R gleich C₁-C₈-Alkyl (bevorzugt) oder C₃-C₈-Cycloalkyl
- R⁵: H oder C₁-C₈-Alkyl,
- R⁶: Aryl, mit bis zu drei C₁-C₈-Alkyl- oder C₁-C₈-Alkoxy substituiertes Aryl, oder C₃-C₈-Cycloalkyl
sind,
als UV-Filter (vorzugsweise UV-B-Filter) in kosmetischen oder dermatologischen Formulierungen.

Dabei gilt die Maßgabe, dass
R⁵ gleich H ist, wenn R⁴ COR ist.

Im Rahmen dieses Textes werden Verbindungen anhand Ihrer Formelbilder angegeben; unabhängig von der im Einzelfall gewählten bildlichen Darstellung umfasst jedes Formelbild dabei sämtliche möglichen Konfigurationsisomeren der angegebenen Verbindung sowie deren Mischungen; insbesondere sind die möglichen E/Z-Isomeren (sowie deren Mischungen) mit umfasst, im Falle der Anwesenheit chiraler Zentren sind jeweils die R- und die S-Enantiomere (sowie deren Mischungen) mit umfasst.

Dieser erfindungsgemäßen Verwendung entspricht ein Verfahren zur Herstellung einer kosmetischen oder dermatologischen Formulierung, wobei eine wirksame Menge einer oder mehrerer Verbindungen der Formel I (wie vorstehend definiert) mit weiteren Bestandteilen einer kosmetischen oder dermatologischen Formulierung vermischt wird.

Der erfindungsgemäßen Verwendung entspricht ferner ein Verfahren zum Schutz von Haut oder Haar gegen UV-Strahlung, insbesondere UV-B-Strahlung, wobei eine wirksame Menge einer oder mehrerer Verbindungen der Formel I (wie vorstehend definiert) in Form einer kosmetischen oder dermatologischen Formulierung, die weitere Bestandteile enthält, auf die Haut oder das Haar appliziert wird.

Die erfindungsgemäß zu verwendenden Verbindungen der Formel I sind insbesondere zur Kombination mit UV-Filtern aus der Gruppe der Methoxycinnamat-Derivate und/oder Dibenzoylmethan-Derivate geeignet; solche Kombinationen haben sich als überraschend photostabil erwiesen.

Erfindungsgemäße Verbindungen sind zwar in der DE 10 87 902 A1 in Form einer (sehr breiten) allgemeinen Formel beschrieben, allerdings nur für den technischen UV-Schutz, so dass hinsichtlich des jeweiligen Fachgebietes und der jeweiligen Bedürfnisse deutliche Unterschiede bestehen. So ist die Einarbeitung (und Fixierung) eines technischen UV-Filters in Kunststoffe, Folien, Fasern oder dergleichen etwas gänzlich anderes als die Einarbeitung in eine kosmetische Formulierung (z.B eine O/W-Emulsion), die auf die Haut aufgetragen wird und - abhängig von der chemischen Struktur des UV-Filters zu Hautpenetration führen kann.

In der DE 19755650 A1 werden substituierte alpha-Methyl-Styrolderivate offenbart, die als photostabile UV-Filter eingesetzt werden können. Die offenbarten Verbindungen umfassen dabei immer eine alpha-Methyl-Gruppe und zwei identische endständige Gruppen (als R³ bzw. R⁴ bezeichnet). Die offenbarten Verbindungen sind im Unterschied zu den erfindungsgemäß einzusetzenden Verbindungen UV-A-Filter.

Es war somit überraschend, daß die erfindungsgemäßen Verbindungen der Formel I hervorragende UV-B-Filter sind und in ganz besonderer Weise photostabile Mischungen (mit anderen UV-Filtern) ergeben.

Besonders bevorzugt zur Verwendung als UV-Filter in kosmetischen oder dermatologischen Formulierungen sind Verbindungen der obigen Formel I, wobei
- R¹-R³: unabhängig voneinander Wasserstoff, C₁-C₈-Alkyl oder C₁-C₈-Alkoxy,
- R⁴: CO₂R mit R gleich C₁-C₈-Alkyl,
- R⁵: H oder C₁-C₈-Alkyl,
- R⁶: Aryl oder mit bis zu drei C₁-C₈-Alkyl- oder C₁-C₈-Alkoxy substituiertes Aryl
sind.

Im Unterschied zu den Verbindungen mit R⁴ gleich COR oder CONR₂ sind die genannten bevorzugt einzusetzenden Verbindungen in kosmetischen Ölen recht gut löslich. Während die Löslichkeit der bevorzugt einzusetzenden Verbindungen in kosmetischen Ölen wie z.B. Isopropylmyristat, Miglyol-812 oder Witconol TN regelmäßig größer ist als 10 Gew.-%, bezogen auf die Gesamtmasse der resultierenden Mischung (Lösung), ist die Löslichkeit für die Verbindungen mit R⁴ gleich COR oder CONR₂ regelmäßig niedriger als 10 Gew.-%.

Besonders bevorzugt ist die Verwendung von Verbindungen der Formel I, wobei
- R¹: Methoxy,
- R² und R³: H,
- R⁴: CO₂R mit R gleich C₂-C₅-Alkyl und
- R⁶: Phenyl oder mit bis zu drei C₁-C₈-Alkyl- oder C₁-C₈-Alkoxy substituiertes Phenyl
sind.

Der Einsatz dieser Verbindungen führt zu besonders photostabilen Mischungen, vergleiche dazu auch die Beispiele unten.

Wiederum aus Gründen einer besonders guten Löslichkeit ist die Verwendung von Verbindungen besonders bevorzugt, in denen
- R¹: Methoxy, das in para-Position zu dem die Substituenten R₄, R₅ und R₆ tragenden Rest angeordnet ist,
- R² und R³: Wasserstoff,
- R⁴: CO₂R mit R gleich C₄-C₅-Alkyl (vorzugsweise n-Butyl),
- R⁵: H oder C₁-C₈-Alkyl, und
- R⁶: Phenyl
sind.

Im Unterschied zu den ansonsten identischen Verbindungen mit R gleich C₁-C₃-Alkyl sind die mit R gleich C₄-C₅-Alkyl Flüssigkeiten (und keine Feststoffe), so dass bei Einsatz der besonders bevorzugten Verbindungen keine Gefahr des Auskristallisierens in der fertigen kosmetischen oder dermatologischen Formulierung besteht.

Zur Verwendung als UV-Filter für den UV-B-Bereich sind folgende Verbindungen bevorzugt:

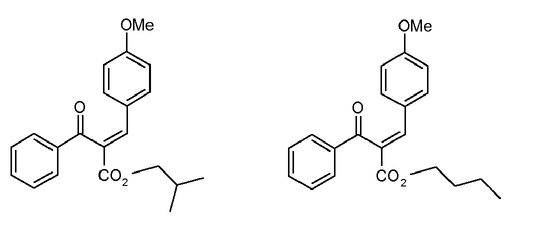

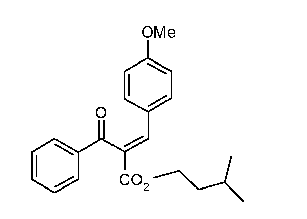

Besonders bevorzugt zur Verwendung als UV-Filter in kosmetischen oder dermatologischen Formulierungen sind auch Verbindungen der obigen Formel I, wobei R⁶ gleich C₃-C₈-Cycloalkyl, vorzugsweise Cyclohexyl ist. Bei ihrer Verwendung ergeben sich besonders photostabile Formulierungen.

Hierbei ist dann vorzugsweise, insbesondere zum Erreichen einer guten Löslichkeit R⁴ gleich CO₂R mit R gleich C₁-C₈-Alkyl, vorzugsweise R gleich Methyl oder Ethyl. Bei R gleich Ethyl ist die Löslichkeit besonders gut.

Die erfindungsgemäß zu verwendenden Benzyliden-β-dicarbonylverbindungen können als UV-Filter (UV-Absorber) in kosmetischen oder dermatologischen Formulierungen, insbesondere zum Schutz vor akuten (Sonnenbrand) sowie chronischen (frühzeitiger Hautalterung) Hautschäden besonders in Sonnenschutzmitteln, Tagespflegeprodukten und Haarpflegeprodukten verwendet werden.

Die erfindungsgemäß zu verwendenden Benzyliden-β-dicarbonylverbindungen können einzeln oder in Mischung in den entsprechenden Formulierungen (Zubereitungen) eingesetzt werden; man kann sie auch in Kombination mit UV-Absorbern anderer Substanzklassen oder auch mit diesen in beliebigen Gemischen untereinander einsetzen.

Eine erfindungsgemäße kosmetische oder dermatologische Formulierung (Zubereitung) umfasst
- eine oder mehrere Verbindungen der Formel I wie oben definiert (mit der allgemeinen oder einer bevorzugten Bedeutung der Substituenten), als UV-Absorber.

Eine bevorzugte kosmetische oder dermatologische Formulierung umfasst weiter:
- einen oder mehrere weitere UV-Absorber, insbesondere aus der Gruppe der Methoxycinnamat-Derivate (p-Methoxyzimtsäureester) und/oder Dibenzoylmethan-Derivate
   und/oder
- umhüllte oder nicht umhüllte Pigmente von Metalloxiden.

Diese Aspekte werden nachfolgend noch im Detail erläutert, wobei auch besonders bevorzugte Ausgestaltungen angegeben werden. In den kosmetischen oder dermatologischen Formulierungen sind die eingesetzten UV-Absorber bzw. Pigmente vorzugsweise so ausgewählt und in ihrer jeweiligen Menge so aufeinander abgestimmt, dass sie derart zusammenwirken, dass der Sonnenschutzfaktor der Formulierung synergistisch erhöht ist.

Besonders vorteilhaft sind die eingesetzten UV-Absorber so ausgewählt und in ihrer jeweiligen Menge so aufeinander abgestimmt, dass die kritische Wellenlänge der Formulierung λ_{crit.}>380 nm ist. Die kritische Wellenlänge ist dabei die Wellenlänge, bei das Integral der spektralen Absorptionskurve 90% des Integrals von 290-400 nm erreicht.

Beispielhaft seien die folgenden UV-Absorber genannt, mit denen die Verbindungen der Formel I kombiniert werden können:
- p-Aminobenzoesäure
- p-Aminobenzoesäureethylester (25 Mol) ethoxyliert
- p-Dimethylaminobenzoesäure-2-ethylhexylester
- p-Aminobenzoesäureethylester (2 Mol) N-propoxyliert
- p-Aminobenzoesäureglycerinester
- Salicylsäure-homomenthylester (Homosalate) (Neo Heliopan^{®}HMS)
- Salicylsäure-2-ethylhexylester (Neo Heliopan^{®}OS)
- Triethanolaminsalicylat
- 4-Isopropylbenzylsalicylat
- Anthranilsäurementhylester (Neo Heliopan^{®}MA)
- Diisopropylzimtsäureethylester
- p-Methoxyzimtsäure-2-ethylhexylester (Neo Heliopan^{®}AV)
- Diisopropylzimtsäuremethylester
- p-Methoxyzimtsäureisoamylester (Neo Heliopan^{®}E 1000)
- p-Methoxyzimtsäure-diethanolaminsalz
- p-Methoxyzimtsäure-isopropylester
- 2-Ethylhexyl-2-cyano-3,3-diphenylacrylat (Neo Heliopan^{®}303)
- Ethyl-2-cyano-3,3'-diphenylacrylat
- 2-Phenylbenzimidazolsulfonsäure und Salze (Neo Heliopan^{®}Hydro)
- 3-(4'-Trimethylammonium)-benzyliden-bornan-2-on-methylsulfat
- Terephthalyliden-dibornansulfonsäure und Salze (Mexoryl^{®}SX)
- 4-t-Butyl-4'-methoxy-dibenzoylmethan (Avobenzon) / (Neo Heliopan^{®}357)
- ß-Imidazol-4(5)-acrylsäure (Urocaninsäure)
- 2-Hydroxy-4-methoxybenzophenon (Neo Heliopan^{®}BB)
- 2-Hydroxy-4-methoxybenzophenon-5-sulfonsäure
- Dihydroxy-4-methoxybenzophenon
- 2,4-Dihydroxybenzophenon
- Tetrahydroxybenzophenon
- 2,2'-Dihydroxy-4,4'-dimethoxybenzophenon
- 2-Hydroxy-4-n-octoxybenzophenon
- 2-Hydroxy-4-methoxy-4'-methylbenzophenon
- 3-(4'-Sulfo)benzyliden-bornan-2-on und Salze
- 3-(4'-Methylbenzyliden)-d,l-campher (Neo Heliopan^{®}MBC)
- 3-Benzyliden-d,l-campher
- 4-Isopropyldibenzoylmethan
- 2,4,6-Trianilino-(p-carbo-2'-ethylhexyl-1'-oxy)-1,3,5-triazin
- Phenylen-bis-benzimidazyl-tetrasulfonsäure-dinatriumsalz (Neo Heliopan^{®}AP)
- 2,2'-(1 ,4-Phenylen)-bis-(1 H-benzimidazol-4,6-disulfonsäure), Mononatriumsalz
- N-[(2 und 4)-[2-(oxoborn-3-yliden)methyl]benzyl]-acrylamid-Polymer
- Phenol, -(2H-benzotriazol-2-yl)-4-methyl-6-(2-methyl-3(1,3,3,3-tetramethyl-1-(trimethylsilyl)-oxy)-disiloxyanyl)-propyl), (Mexoryl^{®}XL)
- 4,4'-[(6-[4-(1,1-Dimethyl)-aminocarbonyl)-phenylamino]-1,3,5-triazin-2,4-diyl)diimino]-bis-(benzoesäure-2-ethylhexylester) (Uvasorb^{®}HEB)
- 2,2'-Methylen-bis-(6-(2H-benztriazol-2-yl)-4-1,1,3,3-tetramethylbutyl)-phenol), (Tinosorb^{®}M)
- 2,4-bis-[4-(2-ethylhexyloxy)-2-hydroxyphenyl]-1,3,5-triazin
- Benzylidenmalonat-Polysiloxan (Parsol^{®}SLX)
- Glyceryl-ethylhexanoat-dimethoxycinnamat
- Dinatrium-2,2'-dihydroxy-4,4'-dimethoxy-5,5'-disulfo-benzophenon
- Dipropylenglykolsalicylat
- Natrium-hydroxymethoxybenzophenon-sulfonat
- 4,4',4-(1,3,5-Triazin-2,4,6-triyltriimino)-tris-benzoesäure-tris(2-ethylhexylester) (Uvinul^{®}T150)
- 2,4-Bis-[{(4-(2-Ethyl-hexyloxy)-2-hydroxy}-phenyl]-6-(4-methoxyphenyl)-1,3,5-triazin, (Tinosorb^{®}S)
- 2,4-Bis-[{(4-(3-sulfonato)-2-hydroxy-propyloxy)-2-hydroxy}-phenyl]-6-(4-methoxyphenyl)-1,3,5-triazin-Natriumsalz
- 2,4-Bis-[{(3-(2-Propyloxy)-2-hydroxy-propyloxy)-2-hydroxy}-phenyl]-6-(4-methoxy-phenyl)-1,3,5-triazin
- 2,4-Bis-[{4-(2-Ethyl-hexyloxy)-2-hydroxy}-phenyl]-6-[4-(2-methoxyethyl-carbonyl)-phenylamino]-1,3,5-triazin
- 2,4-Bis-[{4-(3-(2-propyloxy)-2-hydroxy-propyloxy)-2-hydroxy}-phenyl]-6-[4-(2-ethylcarboxyl)-phenylamino]-1,3,5-triazin
- 2,4-Bis-[{4-(2-Ethyl-hexyloxy)-2-hydroxy}-phenyl]-6-(1-methyl-pyrrol-2-yl-)-1,3,5-triazin
- 2,4-Bis-[{4-tris-(trimethylsiloxy-silylpropyloxy)-2-hydroxy}-phenyl]-6-(4-methoxyphenyl)-1,3,5-triazin
- 2,4-Bis-[{4-(2"-Methylpropenyloxy)-2-hydroxy}-phenyl]-6-(4-methoxyphenyl)-1,3,5-triazin
- 2,4-Bis-[{4-(1',1',1',3'5',5',5'-Heptamethylsiloxy-2"-methyl-propyloxy)-2-hydroxy}-phenyl]-6-(4-methoxyphenyl)-1,3,5-triazin
- 2-(4-Diethylamino-2-hydroxybenzoyl)-benzoesäure-hexylester (Uvinul^{®} A Plus)
- Indanylidenverbindungen gemäß DE 100 55 940 (= WO 02/38537)

Besonders zur Kombination geeignete UV-Absorber sind dabei
- p-Aminobenzoesäure
- 3-(4'-Trimethylammonium)-benzyliden-bornan-2-on-methylsulfat
- Salicylsäure-homomenthylester (Neo Heliopan^{®}HMS)
- 2-Hydroxy-4-methoxy-benzophenon (Neo Heliopan^{®}BB)
- 2-Phenylbenzimidazolsulfonsäure (Neo Heliopan^{®}Hydro)
- Terephthalyliden-dibornansulfonsäure und Salze (Mexoryl^{®}SX)
- 4-tert.-Butyl-4'-methoxydibenzoylmethan (Neo Heliopan^{®}357)
- 3-(4'-Sulfo)benzyliden-bornan-2-on und Salze
- 2-Ethylhexyl-2-cyano-3,3-diphenylacrylat (Neo Heliopan^{®}303)
- N-[(2 und 4)-[2-(oxoborn-3-yliden)methyl]benzyl]-acrylamid-Polymer
- p-Methoxyzimtsäure-2-ethylhexylester (Neo Heliopan^{®}AV)
- p-Aminobenzoesäure-ethylester (25 Mol) ethoxyliert
- p-Methoxyzimtsäure-isoamylester (Neo Heliopan^{®}E1000)
- 2,4,6-Trianilino-(p-carbo-2'-ethylhexyl-1'-oxy)-1,3,5-triazin (Uvinul^{®}T150)
- Phenol, 2-(2H-benzotriazol-2-yl)-4-methyl-6-(2-methyl-3(1,3,3,3-tetramethyl-1-(trimethylsilyl)-oxy)-disiloxyanyl)-propyl), (Mexoryl^{®}XL)
- 4,4'-[(6-[4-(1,1-Dimethyl)-aminocarbonyl)-phenylamino]-1,3,5-triazin-2,4-diyl)-diimino]-bis-(benzoesäure-2-ethylhexylester), (UvasorbHEB)
- 3-(4'-Methylbenzyliden)-d,l-campher (Neo Helipan^{®}MBC)
- 3-Benzylidencampher
- Salicylsäure-2-ethylhexylester (Neo Helipan^{®}OS)
- 4-Dimethylaminobenzoesäure-2-ethylhexylester (Padimate O)
- Hydroxy-4-methoxy-benzophenon-5-sulfonsäure und Na-Salz
- 2,2'-Methylen-bis-(6-(2H-benztriazol-2-yl)-4-1,1,3,3-tetramethylbutyl)-phenol), (Tinosorb^{®}M)
- Phenylen-bis-benzimidazyl-tetrasulfonsäure-dinatriumsalz (Neo Heliopan^{®}AP)
- 2,4-Bis-[{(4-(2-Ethyl-hexyloxy)-2-hydroxy}-phenyl]-6-(4-methoxyphenyl)-1,3,5-triazin, (Tinosorb^{®}S)
- Benzylidenmalonat-Polysiloxan (Parsol^{®}SLX)
- Menthylanthranilat (Neo Heliopan^{®}MA)
- 2-(4-Diethylamino-2-hydroxybenzoyl)-benzoesäure-hexylester (Uvinul^{®} A Plus)
- Indanylidenverbindungen gemäß DE 100 55 940 (= WO 02/38537)

Es kann auch von Vorteil sein, polymer-gebundene oder polymere UV-Absorber in erfindungsgemäßen Formulierungen zu verwenden, insbesondere solche, wie sie in WO-A-92/20690 beschrieben werden. Ebenso ist die Kombination der erfindungsgemäß zu verwendenden Benzyliden-β-dicarbonylverbindungen mit feinteiligen anorganischen und organischen Pigmenten, wie z.B. Titandioxid, Zinkoxid und Eisenoxid bzw. Tinosorb^{®}M, in Sonnenschutz- und Tagespflegeprodukten mit UV-Schutz möglich.

Die Aufzählung der genannten UV-Filter, die im Sinne der vorliegenden Erfindung in Kombination mit den Benzyliden-β-dicarbonylverbindungen der Formel I eingesetzt werden können, ist selbstverständlich nicht abschließend.

Die Gesamtmenge aller (einfach oder mehrfach) sulfonierten wasserlöslichen UV-Filtersubstanzen in den fertigen kosmetischen oder dermatologischen Formulierungen, beispielsweise an Phenylen-bis-benzimidazyl-tetrasulfonsäure-dinatriumsalz bzw. deren Salzen und/oder die entsprechende Disulfonsäure bzw. deren Salzen und/oder 2-Phenylbenzimidazol-5-sulfonsäure bzw. deren Salzen und/oder 2-Hydroxy-4-meth-oxybenzophenon-5-sulfonsäure bzw. deren Salzen und/oder 4-(2-Oxo-3-bornyli-denmethyl)-benzolsulfonsäure bzw. deren Salzen und/oder 2-Methyl-5-(2-oxo-3-bornyliden-methyl)-benzolsulfonsäure bzw. deren Salzen und/oder Benzol-1,4-di-(2-oxo-3-bornylidenmethyl)-10-sulfonsäure bzw. deren Salzen, wird vorteilhaft aus dem Bereich von 0,1 bis 10,0 Gew.-%, bevorzugt 0,5 bis 6,0 Gew.-% gewählt, bezogen auf das Gesamtgewicht der Formulierungen, falls die Gegenwart dieser Substanzen erwünscht ist.

Die Gesamtmenge an öllöslichen UV-Filtersubstanzen (einschließlich der Verbindungen der Formel I) in den fertigen kosmetischen oder dermatologischen Formulierungen, beispielsweise an 4,4',4"-(1,3,5-Triazin-2,4,6-triyltriimino)-tris-benzoesäure-tris-(2-ethylhexylester) und/oder 4-tert.-Butyl-4'-methoxy-dibenzoylmethan und/oder 4-Methylbenzylidencampher und/oder Octyldimethylp-Aminobenzoesäure und/oder Mexoryl^{®}XL und/oder Uvasorb^{®}HEB und/oder Tinosorb^{®}S und/oder Benzophenon-3 und/oder Parsol^{®}SLX und/oder Neo Heliopan^{®}MA wird vorteilhaft aus dem Bereich von 0,1 bis 10,0 Gew.-%, bevorzugt 0,5 bis 6,0 Gew.-% gewählt, bezogen auf das Gesamtgewicht der Formulierungen, falls die Gegenwart dieser Substanzen erwünscht ist.

Die Gesamtmenge an 2-Ethylhexyl-p-methoxy-cinnamaten und/oder p-Methoxyzimtsäureisoamylester in den fertigen kosmetischen oder dermatologischen Zubereitungen wird vorteilhaft aus dem Bereich von 0,1 bis 15,0 Gew.-%, bevorzugt 0,5 bis 7,5 Gew.-% gewählt, bezogen auf das Gesamtgewicht der Formulierungen, falls die Gegenwart dieser Substanzen erwünscht ist.

Die Gesamtmenge an Ethylhexyl-2-cyano-3,3-diphenylacrylat in den fertigen kosmetischen oder dermatologischen Formulierungen wird, falls die Gegenwart dieser Substanz erwünscht ist, vorteilhaft aus dem Bereich von 0,1 bis 15,0 Gew.-%, bevorzugt 0,5 bis 10,0 Gew.-% gewählt, bezogen auf das Gesamtgewicht der Formulierungen.

Die Gesamtmenge an einem oder mehreren Salicylsäurederivaten in den fertigen kosmetischen oder dermatologischen Formulierungen wird in vielen Fällen vorteilhaft aus dem Bereich von 0,1 bis 15,0 Gew.-%, bevorzugt 0,5 bis 10,0 Gew.-% gewählt, bezogen auf das Gesamtgewicht der Formulierungen. Wenn Ethylhexylsalicylat gewählt wird, ist es von Vorteil, dessen Gesamtmenge aus dem Bereich von 0,1 bis 5,0 Gew.-% zu wählen. Wenn Homomenthylsalicylat gewählt wird, ist es von Vorteil, dessen Gesamtmenge aus dem Bereich von 0,1 bis 10,0 Gew.-% zu wählen.

Die besonders bevorzugten Kombinationen von (a) p-Methoxyzimtsäureestern (Methoxycinnamate) und/oder Dibenzoylmethanderivaten und (b) Verbindungen der Formel I lassen sich lichtstabil formulieren durch Einsatz von z.B. 0,1 bis 5 Gew.-%, bevorzugt 1 bis 3 Gew.-% 4-tert.-Butyl-4'-methoxydibenzoylmethan, 0,1 bis 10 Gew.-%, bevorzugt 1 bis 7,5 Gew.-% p-Methoxyzimtsäureethylhexyl- oder isoamylester und mindestens 0,2 Gew.-%, bevorzugt 1 bis 6 Gew.-% der erfindungsgemäß einzusetzenden Verbindungen der Formel I.

Bevorzugt wird ein Verhältnis eingestellt im Bereich 1 Teil Dibenzoylmethanderivat, 2,5-3,5 Teile p-Methoxyzimtsäureester und 1,5-2,5 Teile der erfindungsgemäß einzusetzenden Benzyliden-β-dicarbonylverbindungen. Besonders bevorzugt ist die Kombination von 1 Teil Dibenzoylmethanderivat, 3 Teile p-Methoxyzimtsäureester und 2 Teilen der erfindungsgemäß einzusetzenden Benzyliden-ß-dicarbonylverbindungen.

Vorteilhaft ist es zudem, zu dieser Dreierkombination einen oder weitere sehr photostabile UV-Absorber, wie z.B. Methylbenzylidencampher, 2-Ethylhexyl-2-cyano-3,3'-diphenylacrylat, Octyltriazon, Uvasorb^{®}HEB, Tinosorb^{®}S, Tinosorb^{®}M, Ethylhexylsalicylat, Homomenthylsalicylat sowie Phenylenbenzimidazolsulfonsäure oder Phenylen-bis-benzimidazoltetrasulfonsäure-dinatriumsalz, Mexoryl^{®}SX, Mexoryl^{®}XL, Parsol^{®}SLX, Uvinul^{®}A Plus oder Indanylidenverbindungen gemäß DE 100 55 940 hinzuzusetzen.

In kosmetischen oder dermatologische Formulierungen wird überraschenderweise durch die Verwendung von Benzyliden-ß-dicarbonylverbindungen der Formel I in Kombination mit anderen UV-Filtern eine synergistische Erhöhung des Sonnenschutzfaktors erreicht. Beispiel für eine synergistische Erhöhung des Sonnenschutzfaktors sind kosmetische oder dermatologische Emulsionen, die sowohl eine Verbindung der Formel I als auch Etylhexylmethoxycinnamat oder Octocrylene, oder eine Kombination einer Verbindung der Formel I mit Ethylhexylmethoxycinnamat und 2-Phenylbenzimidazolsulfonsäure, oder Ethylhexyl-methoxycinnamat und Methylbenzylidencampher, oder Ethylhexyl-methoxycinnamat und 4-t-Butyl-4'-methoxy-dibenzoylmethan, oder Neo Heliopan^{®}AP und Ethylhexylmethoxycinnamat, oder eine Kombination von Verbindung der Formel I mit Octocrylene, Methylbenzylidencampher und Zinkoxid enthält. Ebenfalls weisen Kombinationen von Verbindung der Formel I mit Dibenzoylmethanen, Methylbenzylidencampher, 2-Phenylbenzimidazolsulfonsäure, Neo Heliopan^{®}AP, Mexoryl^{®}SX, Mexoryl^{®}XL, Parsol^{®}SLX, Tinosorb^{®}S, Tinosorb^{®}M, Uvinul^{®}T150, Uvasorb^{®}HEB, Uvinul^{®}A Plus oder Indanylidenverbindungen gemäß DE 100 55 940 sowie mikrofeinen Pigmenten, Zinkoxid und Titandioxid, synergistische Erhöhungen des Sonnenschutzfaktors auf. Die genannten UV-Filter-Kombinationen sind hier nur beispielhaft angeführt; ein synergistischer Effekt tritt auch bei Kombination mit anderen UV-Filtern auf. So können einzeln oder in Kombination miteinander alle oben bereits genannten besonders geeigneten UV-Absorber (UV-Filter) sowie alle in den nachfolgenden Veröffentlichungen zugelassenen UV-Filter in Kombination mit Verbindungen der Formel I eingesetzt werden.
- USA:: Food and Drug Administration (FDA). Veröffentlichung in Monographie für Sunscreen Drug Products for Over-The-Counter Human Use.
- Europa:: Richtlinie 76/768 EWG des Rates zur Angleichung der Rechtsvorschriften der Mitgliedsstaaten über kosmetische Mittel an den technischen Fortschritt. Veröffentlichungen im Official Journal of European Communities.
- Japan:: Veröffentlichung der Kosmetik-Richtliniedes Ministry of Health and Welfare (MHW).
- Deutschland:: Veröffentlichung in der Verordnung über kosmetische Mittel gemäß dem Lebensmittel- und Bedarfsgegenstände-Gesetz (LMBG).
- Australien:: Registrierung durch Therapeutic Goods Administration (TGA) und Veröffentlichung im Australian Register of Therapeutic Goods (ARTG).

Regelmäßig wird durch die genannten Kombinationen eine synergistische Erhöhung des UV-Sonnenschutzfaktors erzielt.

Die Kombination von Verbindungen der Formel I mit UV-A-Absorbern, ergibt einen optimalen Breitbandschutzleistung (290-400nm). Insbesondere eine Kombination von Verbindungen der Formel I mit Neo Heliopan^{®} AP (UV-All-Absorber) und Indanylidenverbindungen gemäß DE 100 55 940 (UV-AI-Absorber) ist für diese breite UV-Schutzleistung zu nennen. Weitere UV-A-Filter, die in Kombination mit Verbindungen der Formel I allein oder in Kombination von Verbindungen der Formel I und Neo Heliopan^{®}AP und /oder Indanylidenverbindungen gemäß DE 100 55 940 bevorzugt werden, sind Mexoryl^{®}SX, Mexoryl^{®}XL, Tinosorb^{®}M Tinosorb^{®}S, Benzophenon-3, Benzophenon-4, Neo Heliopan^{®}357, Neo Heliopan^{®}MA und Uvinul^{®}A Plus.

Durch Kombination von Verbindungen der Formel I mit Neo Heliopan^{®}AP und einem UV-B-Filter, z.B. Etylhexylmethoxycinnamat oder UV-B-Filtergemischen sowie beschichteten oder unbeschichteten feindispersen Metalloxiden wie z.B. Zinkoxid, Titandioxid wird eine UV-Breitband-Schutzleistung mit einer kritischen Wellenlänge λ_{crit.}> 380 nm erzielt (vgl. hierzu Diffey in Int. J. Cosm. Science 16, 47 (1994)).

Einige der erfindungsgemäß zu verwendenden Benzyliden-ß-dicarbonylverbindungen der Formel I sind bei Standardbedingungen (25°C; 1013 mbar) (kristalline) Feststoffe und müssen daher in kosmetischen Formulierungen ausreichend gelöst werden, um das Problem der Rekristallisation nach längerer Lagerungszeit zu vermeiden (vgl. dazu die obigen Anmerkungen zu besonders bevorzugten, da flüssigen, Verbindungen der Formel I). Vorteilhafterweise wird zur Vermeidung der Rekristallisation eine ausreichende Menge der üblicherweise in kosmetischen Formulierungen eingesetzten Ölkomponenten, flüssigen öllöslichen UV-Absorber oder Alkohole eingesetzt, z. B. Ethanol, Isopropanol oder 1-Butanol. Besonders bevorzugt ist der Einsatz folgender Ölkomponenten und/oder UV-Absorber zur Erzielung einer ausreichenden Löslichkeit kristalliner erfindungsgemäß zu verwendender Benzyliden-ß-dicarbonylverbindungen der Formel I:

Ethylhexylmethoxycinnamat, Isoamylmethoxycinnamat, Octocrylen, Ethylhexylsalicylat, Homosalat, Menthylanthranilat, Padimate O, Düsopropyladipat, C₁₂₋₁₅-Alkylbenzoat (Witconol TN), Butylenglykoldicaprylat/-dicaprat (Miglyol 8810), Cocoglyceride (Myritol 331), Capryl/capr.-triglyceride (Miglyol 812), Cetearylisononanat (Cetiol SN), PVP/Hexadecen-Copolymer (Unimer U151), Adipinsäure-/Diethylenglykol/Isononansäure-Copolymer (Lexorez 100), Propylenglykoldicapry-lat/ Dicaprat (Myritol PC), Hexyllaurat (Cetiol A), Dicaprylether (Cetiol OE), Diethylhexylnaphthalat (Hallbrite^{®}TQ), Butyloctylsalicylat (Hallbrite^{®}BHB), Dibutyladipat (Cetiol B), Triethylcitrat (Hydagen CAT), Propylenglykoldibenzoat (Finsolv PG 22), Tributylcitrat, Dioctylmalat (Ceraphyl 45), Dipropylenglykol-dibenzoat (Benzoflex 245), Acetyltributylcitrat (Citroflex A-4), Acetyltriethylcitrat (Citroflex A-2). Die Aufzählung der genannten Öle, die im Sinne der vorliegenden Erfindung eingesetzt werden können, ist selbstverständlich nicht abschließend.

Die gesamte Einsatzmenge sämtlicher Komponenten der Ölphase in kosmetischen Emulsionen mit Verbindungen der Formel I liegt vorzugsweise im Bereich von 0,5 bis 30%, bevorzugt 2 bis 15%. Alle oben genannten Ölkomponenten bzw. flüssigen öllöslichen UV-Filter sind ausgezeichnete Lösungsmittel für alle kristallinen öllöslichen UV-Absorber.

Sehr nachteilig ist, wenn UV-Absorber auf Kleidungsstücken nicht mehr auswaschbare Flecken hinterlassen. Insbesondere ist von dem UV-A-Absorber tert.-Butylmethoxydibenzoylmethan bekannt, dass er nicht mehr auswaschbare Flecken auf Textilien erzeugt. Diesen Nachteil haben die erfindungsgemäß zu verwendenden Benzyliden-β-dicarbonylverbindungen nicht, da etwaige Flecken auf Textilien sehr gut auswaschbar sind.

Sonnenschutzprodukte sollen wasserfest sein, damit ein ausreichender UV-Schutz für den Anwender, insbesondere Kinder, beim Schwimmen oder Baden gewährleistet ist. Die erfindungsgemäß zu verwendenden Verbindungen erfüllen diese Anforderungen in besonderem Maße. In einer O/W-Emulsion mit 3% einer Verbindung der erfindungsgemäßen Benzyliden-ß-dicarbonylverbindungen wurden 97% Substantivität des UV-Absorbers nach dem Waschen gemessen und in einer W/O-Emulsion 95%. Weiterhin kann die Wasserfestigkeit von Sonnenschutzprodukten mit wasserlöslichen, einfach oder mehrfach sulfonierten UV-Filtern wie z.B. Neo Heliopan^{®}AP, Mexoryl^{®}SX, Benzophenon-4, Neo Heliopan^{®}Hydro und/oder den oben aufgeführten öllöslichen UV-Absorbern durch Kombination mit Verbindungen der Formel I signifikant erhöht werden.

Es kann ferner von erheblichem Vorteil sein, die erfindungsgemäß genannten UV-Absorber mit chelatisierenden Substanzen, wie sie z.B. in EP-A 496 434, EP-A 313 305 und WO-94/04128 aufgeführt sind, oder mit Polyasparaginsäure und Ethylendiamin-tetramethyl-phosphonsäuresalzen zu kombinieren.

Gegenstand der Erfindung ist ferner die Verwendung der erfindungsgemäß zu verwendenden Verbindungen der Formel I in Kombination mit herkömmlichen UV-Absorbern zur Verstärkung des Schutzes vor schädigender UV-Strahlung über das Maß des Schutzes, der bei Einsatz gleicher Gesamtmengen herkömmlicher oder der erfindungsgemäß zu verwendenden UV-Filter allein erzielt wird (synergistischer Effekt).

Die Gesamtmenge an UV-Filtersubstanzen (UV-A-, UV-B- und/ oder Breitbandfilter) in erfindungsgemäßen kosmetischen oder dermatologischen Formulierungen, sei es als Einzelsubstanz oder in beliebigen Gemischen untereinander, liegt vorteilhaft im Bereich von 0,1 bis 30 Gew.-%, bevorzugt 0,1 bis 10,0 Gew.-%, insbesondere 0,5 bis 5,0 Gew.-%, bezogen auf das Gesamtgewicht der Formulierungen.

Erfindungsgemäße kosmetische und dermatologische Formulierungen enthalten ferner vorteilhaft, wenngleich nicht zwingend, anorganische Pigmente auf Basis von feindispersen Metalloxiden und/oder anderen in Wasser schwerlöslichen oder unlöslichen Metallverbindungen, insbesondere der Oxide des Titans (1102), Zinks (ZnO), Eisens (z.B. Fe₂O₃), Zirkoniums (ZrO₂). Siliciums (SiO₂). Mangans (z.B. MnO), Aluminiums (A1₂O₃), Cers (z.B. Ce₂O₃), Mischoxiden der entsprechenden Metalle sowie Abmischungen aus solchen Oxiden. Diese Pigmente sind röntgen-amorph oder nichtröntgenamorph. Besonders bevorzugt handelt es sich um Pigmente auf der Basis von TiO₂.

Röntgenamorphe Oxidpigmente sind Metalloxide oder Halbmetalloxide, welche bei Röntgenbeugungsexperimenten keine oder keine erkenntliche Kristallstruktur erkennen lassen. Oftmals sind solche Pigmente durch Flammenreaktion erhältlich, beispielsweise dadurch, dass ein Metall- oder Halbmetallhalogenid mit Wasserstoff und Luft (oder reinem Sauerstoff) in einer Flamme umgesetzt wird.

In kosmetischen, dermatologischen oder pharmazeutischen Formulierungen werden röntgenamorphe Oxidpigmente als Verdickungs- und Thixotropierungsmittel, Fließhilfsmittel zur Emulsions- und Dispersionsstabilisierung und als Trägersubstanz (beispielsweise zur Volumenerhöhung von feinteiligen Pulvern oder Pudern) eingesetzt. Bekannte und in der kosmetischen oder dermatologischen Galenik oftmals verwendete röntgenamorphe Oxidpigmente sind beispielsweise hochreines Siliciumoxid. Bevorzugt werden hochreine, röntgenamorphe Siliciumdioxidpigmente mit einer Partikelgröße im Bereich von 5 bis 40 nm und einer aktiven Oberfläche (BET) im Bereich von 50 bis 400 m²/g, bevorzugt 150 bis 300 m²/g, wobei die Partikel als kugelförmige Teilchen mit sehr einheitlicher Abmessung anzusehen sind. Makroskopisch sind die Siliciumdioxidpigment als lockere, weiße Pulver erkenntlich. Siliciumdioxidpigment werden im Handel unter dem Namen Aerosil^{®} (CAS-Nr. 7631-85-9) oder Carb-O-Sil vertrieben Vorteilhafte Aerosil^{®}-Typen sind beispielsweise Aerosil^{®}0X50. Aerosil^{®}130, Aerosil^{®}150, Aerosil^{®}200, Aerosil^{®}300, Aerosil^{®}380, Aerosil^{®}MQX 80. Aerosil^{®} MOX 170, Aerosil^{®}COK 84, Aerosil^{®} R 202, Aerosil^{®}R 805, Aerosil^{®}R 812, Aerosil^{®}R 972, Aerosil^{®}R 974, Aerosil^{®}R976.

Erfindungsgemäße kosmetische oder dermatologische Lichtschutzzubereitungen enthalten vorteilhafterweise 0,1 bis 20 Gew.-%, vorteilhaft 0,5 bis 10 Gew.-%, ganz besonders bevorzugt 1 bis 5 Gew.-% röntgenamorphe Oxidpigmente.

Die nicht-röntgenamorphen anorganischen Pigmente liegen erfindungsgemäß vorteilhaft in hydrophober Form vor, d.h., dass sie oberflächlich wasserabweisend behandelt sind. Diese Oberflächenbehandlung kann darin bestehen, dass die Pigmente nach an sich bekannten Verfahren mit einer dünnen hydrophoben Schicht versehen werden. Eines solcher Verfahren besteht beispielsweise darin, dass die hydrophobe Oberflächenschicht nach einer Reaktion gemäß

n TiO2 + m (RO)3Si-R' → n TiO2 (oberfl.)

erzeugt wird. n und m sind dabei nach Belieben einzusetzende stöchiometrische Parameter, R und R' die gewünschten organischen Reste. Beispielsweise in Analogie zu DE-A 33 14 742 dargestellte hydrophobisierte Pigmente sind von Vorteil.

Beispielsweise seien TiO₂-Pigmente genannt, wie sie unter der Handelsbezeichnung T805 von der Firma Degussa vertrieben werden. Bevorzugt sind auch TiO₂/Fe₂O₃-Mischoxide, wie sie beispielsweise unter der Handelsbezeichnung T817 ebenfalls von der Firma Degussa angeboten werden.

Die Gesamtmenge an anorganischen Pigmenten, insbesondere hydrophoben anorganischen Mikropigmenten in den fertigen kosmetischen oder dermatologischen Formulierungen liegt vorteilhaft im Bereich von 0,1 bis 30 Gew.-%, bevorzugt 0,1 bis 10,0, insbesondere 0.5 bis 6.0 Gew.-%, bezogen auf das Gesamtgewicht der Formulierungen.

Die erfindungsgemäßen kosmetischen und/oder dermatologischen Formulierungen können wie üblich zusammengesetzt sein und dem kosmetischen und/oder dermatologischen Sonnenschutz, ferner zur Behandlung, der Pflege und der Reinigung der Haut und/oder der Haare und als Schminkprodukt in der dekorativen Kosmetik dienen. Entsprechend können die erfindungsgemäßen Formulierungen, je nach ihrem Aufbau, beispielsweise verwendet werden als Hautschutzcreme, Reinigungsmilch, Sonnenschutzlotion, Nährcreme, Tages- oder Nachtcreme usw. Es ist gegebenenfalls möglich und vorteilhaft, die erfindungsgemäßen Formulierungen als Grundlage für pharmazeutische Formulierungen zu verwenden. Bevorzugt sind insbesondere solche kosmetischen und dermatologischen Formulierungen, die in der Form eines Hautpflege- bzw. Schminkproduktes vorliegen. Typische Ausgestaltung sind Cremes, Gele, Lotionen, alkoholische und wäßrig/alkoholische Lösungen, Emulsionen oder Stiftpräparate. Diese Mittel können ferner als weitere Hilfs- und Zusatzstoffe milde Tenside, Co-Emulgatoren, Überfettungsmittel, Perlglanzwachse, Konsistenzgeber, Verdickungsmittel, Polymere, Siliconverbindungen, Fette, Wachse, Stabilisatoren, biogene Wirkstoffe, Deowirkstoffe, Antischuppenmittel, Filmbildner, Quellmittel, Hydrotope, Konservierungsmittel, Insektenrepellentien, Bräunungsmittel, künstliche Selbstbräunungsmittel (z.B. Dihydroxyaceton), Solubilisatoren, Parfümöle, Farbstoffe, keimhemmende Mittel und dergleichen enthalten.

Zur Anwendung werden die erfindungsgemäßen kosmetischen und dermatologischen Formulierungen in der für Kosmetika üblichen Weise auf die Haut und/oder die Haare in ausreichender Menge aufgebracht.

Besonders bevorzugt sind solche erfindungsgemäßen kosmetischen und/oder dermatologischen Formulierungen, die in der Form eines kosmetischen Mittels zum Schutz von Haut und Haaren vorliegen. Vorteilhaft können diese zusätzlich zu erfindungsgemäß verwendeten UV-A-, UV-B- und/oder Breitbandfiltern mindestens ein anorganisches Pigment, bevorzugt ein anorganisches Mikropigment, enthalten.

Die erfindungsgemäßen kosmetischen und/oder dermatologischen Formulierungen können kosmetische Hilfsstoffe enthalten, wie sie üblicherweise in solchen Formulierungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate. Als nicht ionische Emulgatoren bzw. Dispergatoren kommen in Frage die Gruppe, die gebildet wird von Polyglyceryl-2-dipolyhydroxystearate (Dehymuls^{®}PGPH), Polyglyceryl-3-düso-stearate (Lameform^{®}TGI), Polyglyceryl-4-isostearate (Isolan^{®}GI 34), Polyglyceryl-3-oleate, Diisostearyl-Polyglyceryl-3-diisostearate (Isolan^{®}PDI), Polyglyceryl-3-methylglucose distearate (Tego Carey^{®}450), Polyglyceryl-3-beeswax (Cera Bellina^{®}), Polyglyceryl-4-caprate (Polyglycerol caprate T2010/90), Polyglyceryl-3-cetylether (Chimexane^{®}NL), Polyglyceryl-3-distearate (Cremophor^{®}GS 32), Polyglyceryl-2-stearate (Hostacerin^{®}DGMS) und Polyglyceryl-polyricineoleate (Admul^{®}WOL 1403) sowie deren Gemischen.

Die jeweils einzusetzenden Mengen an kosmetischen oder dermatologischen Hilfs- und Trägerstoffen und Parfüm können in Abhängigkeit von der Art des jeweiligen Produktes vom Fachmann durch einfaches Ausprobieren leicht ermittelt werden.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. a-Carotin, b-Carotin, Lycopin) und deren Derivate, Chlorogensäure und deren Derivate, Liponsäure und deren Derivate (z.B. Dihydroliponsäure), Aurothioglucose, Propyl-thiouracil und andere Ihiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, y-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Formulierungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Formulierung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Die Lipidphase kann vorteilhaft gewählt werden aus folgender Substanzgruppe:
- Mineralöle, Mineralwachse;
- Öle, wie Triglyceride der Caprin- oder der Caprylsäure, ferner natürlicher Öle wie z.B. Rizinusöl;
- Fette, Wachse und andere natürliche und synthetische Fettkörper, vor zugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxan, Diethylpolysiloxan, Diphenylpolysiloxan sowie Mischformen daraus.

Die Ölphasen der Emulsionen, Oleogele bzw. Hydrodispersionen oder Lipodispersionen im Sinne der vorliegenden Erfindung umfassen vorteilhafterweise Substanzen aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, -palmitat, -stearat, -oleat, n-Butylstearat, n-Hexyllaurat, n-Decyloleat, Isooctylstearat, Iso-nonylstearat, Isononylisononanat, 2-Ethylhexylpalmitat, Ethylhexyllaurat, 2-Hexyl-decylstearat, 2-Octyldodecylpalmitat, Oleyloleat, Oleylerucat, Erucyloleat sowie synthetische, halbsynthetische und natürliche Gemische solcher Ester, zB. Jojobaöl.

Ferner kann die Ölphase vorteilhaft gewählt werden aus der Gruppe der verzweigten und unverzweigten Kohlenwasserstoffe und -wachse, der Siliconöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten können vorteilhaft im Sinne der vorliegenden Erfindung eingesetzt werden. Es kann auch gegebenenfalls vorteilhaft sein, Wachse, beispielsweise Cetylpalmitat, als alleinige (nicht in relevantem Maße UV-absorbierende) Lipidkomponente der Ölphase einzusetzen.

Vorteilhaft umfasst die Ölphase eine oder mehrere Substanzen aus der Gruppe gewählt aus der Gruppe 2-Ethylhexylisostearat, Ocryldodecanol, Isotridecylisononanoat, Isoeicosan, 2-Ethylhexylcocoat, C₁₂₋₁₅-Alkyl-benzoat, Capryl- Caprinsäure-triglycerid, Dicaprylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecyl-isononanoat.

Die Ölphase kann auch vorteilhaft einen Gehalt an cyclischen oder linearen Silikonölen aufweisen, wobei allerdings bevorzugt wird, außer dem Silikonöl oder Siliconölen einen zusätzlichen Gehalt an anderen Ölphasenkomponenten zu verwenden.

Vorteilhaft wird Cyclomethicon (Octamethylcyclotetrasiloxan) als zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat sowie aus Cyclomethicon und 2-Ethylhexylisostearat.

Die wäßrige Phase der erfindungsgemäßen Formulierungen enthält gegebenenfalls vorteilhaft Alkohole, Diole oder Polyole (Niederalkyl), sowie deren Ether, vorzugs-weise Ethanol, Isopropanol, Propylenglykol, Glycerin, Ethylenglykol-monoethyl- oder -monobutylether, Propylenglykolmonomethyl, - monoethyl- oder -monobutylether, Diethylenglykolmonomethyl- oder - monoethylether und analoge Produkte, ferner Alkohole (Niederalkyl), z.B. Ethanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.
Eine umfassende Beschreibung der in kosmetischen Mitteln eingesetzten Roh- und Wirkstoffe ist in DE 199 19 630 A1 dargestellt.

Die Erfindung betrifft auch neue Verbindungen der Formel I 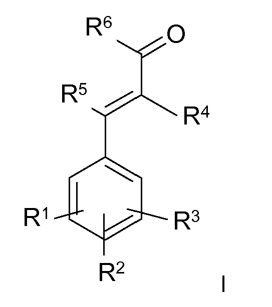 und zwar insbesondere diejenigen, worin
- R¹: C₁-C₈-Alkoxy das in para-Position zu dem die Substituenten R⁴, R⁵ und R⁶ tragenden Rest angeordnet ist, vorzugsweise Methoxy,
- R² und R³: Wasserstoff,
- R⁴: CO₂R mit R gleich C₁-C₈-Alkyl,
- R⁵: H,
- R⁶: Phenyl oder Cyclohexyl
ist.

Bevorzugt sind dabei (a) Verbindungen, in denen
- R⁴: CO₂R mit R gleich Methyl, Ethyl, n-Propyl, iso-Propyl, n-Butyl, sec-Butyl, iso-Butyl, tert-Butyl, iso-Amyl, vorzugsweise n-Butyl,und
- R⁶: Phenyl
ist.

Bevorzugt sind aber auch (b) Verbindung, in denen
- R⁴: CO₂R mit R gleich Methyl, Ethyl, n-Propyl, iso-Propyl, n-Butyl, sec-Butyl, iso-Butyl oder tert-Butyl, vorzugsweise Ethyl, und
- R⁶: Cyclohexyl
ist.

Weitere bevorzugte Ausgestaltungen der Erfindung ergeben sich aus den nachfolgenden Beispielen (inklusive Rezepturbeispielen, Beispielen zur Photostabilität usw.) und den beigefügten Patentansprüchen.

### Beispiele zur Herstellung und zum Absorptionsverhalten:

### Beispiel 1

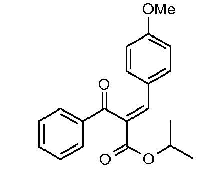

1 Mol Benzoylessigsäure-isopropylester und 1 Mol Anisaldehyd werden in 200g Toluol zusammen gegeben und mit 0,1 Mol Ammoniumacetat sowie 0,1 Mol Propionsäure versetzt. Man erhitzt zum sieden und schleust das entstehende Wasser über einen Wasserabscheider aus. Nach 4h Stunden wird auf RT abgekühlt mit 100g Wasser gewaschen und das Produkt als E/Z-Gemisch (86:14) destilliert. Ausbeute: 60% d.Th. Extinktion: 640 bei 316 nm

### Beispiel 2

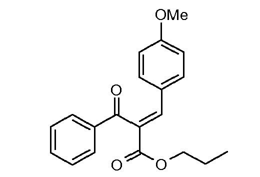

1 Mol Benzoylessigsäure-n-propylester werden analog zu Beispiel 1 umgesetzt. Ausbeute: 60% d.Th. (E/Z-Gemisch 86:14) Extinktion: 660 bei 316 nm

### Beispiel 3

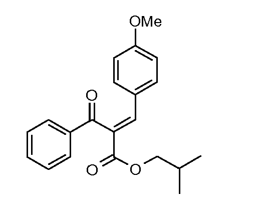

1 Mol Benzoylessigsäure-isobutylester werden analog zu Beispiel 1 umgesetzt. Ausbeute: 60% d.Th. (E/Z-Gemisch 80:20) Extinktion: 640 bei 316 nm

### Beispiel 4

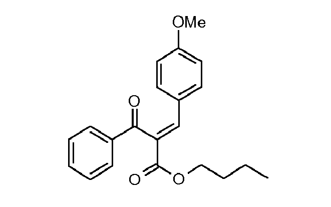

1 Mol Benzoylessigsäure-n-butylester werden analog zu Beispiel 1 umgesetzt. Ausbeute: 60% d.Th. (E/Z-Gemisch 85:15) Extinktion: 660 bei 316 nm

### Beispiel 5

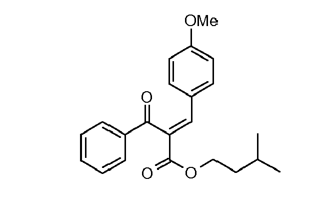

1 Mol Benzoylessigsäure-isoamylester werden analog zu Beispiel 1 umgesetzt. Ausbeute: 60% d.Th. (E/Z-Gemisch 86:14) Extinktion: 630 bei 316 nm

### Beispiel 6

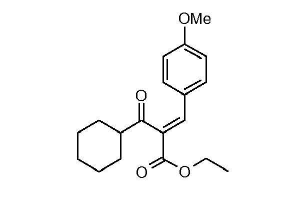

1 Mol 3-Cyclohexyl-3-oxo-propionsäure-ethylester werden analog zu Beispiel 1 umgesetzt. Ausbeute: 60% d.Th. (E/Z-Gemisch 70:30) Extinktion: 700 bei 316 nm

### Beispiel 7

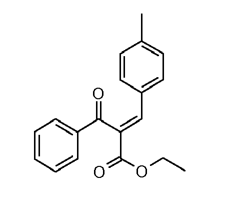

1 Mol Benzoylessigsäure-ethylester werden analog zu Beispiel 1 mit p-Tolylaldehyd umgesetzt. Ausbeute: 60% d.Th. (E/Z-Gemisch 84:16) Extinktion: 690 bei 292 nm

### Beispiel 8

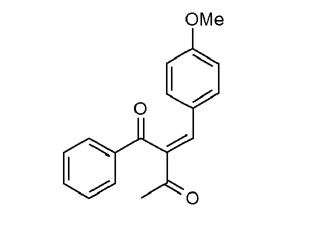

1 Mol Benzoylaceton werden analog zu Beispiel 1 umgesetzt. Ausbeute: 60% d.Th. (E/Z-Gemisch 15:85). Durch Umkristallisation aus Methanol erhält man das reine Z-Isomer als Feststoff (Fp 76°C) Extinktion: 816 bei 323 nm

### Beispiel 9

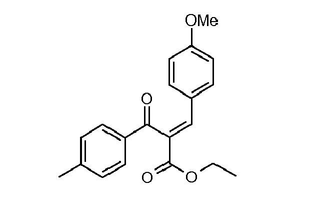

1 Mol p-Methylbenzoyessigsäure-ethylester werden analog zu Beispiel 1 mit Anisaldehyd umgesetzt. Ausbeute: 60% d.Th. (E/Z-Gemisch 86:14) Extinktion: 660 bei 316 nm

### Rezepturbeispiel 1

Sonnenschutz Soft Creme (O/W), in-vitro SPF 3, wasserfest

| **Teil** | **Rohstoffe** | **INCI Bezeichnung** | **% (Gew.)** |
|---|---|---|---|
| **A** | Crodafos MCA | Cetyl Phosphate | 1.50 |
| | Cutina MD | Glyceryl Stearate | 2.00 |
| | Copherol 1250 | Tocopherylacetate | 0.50 |
| | Lanette 16 | Cetyl Alcohol | 1.00 |
| | Tegosoft TN | C 12-15 Alkyl Benzoate | 24.00 |
| | Prisorine 3505 | Isostearic Acid | 1.00 |
| | UV-Filter-Verbindung gemäß Formel I | | 3.00 |
| **B** | Wasser, dest. | Water (Aqua) | 59.60 |
| | EDETA B fl. | Tetrasodium EDTA | 0.20 |
| | Glycerin, 99% | Glycerin | 3.00 |
| | Phenoxyethanol | Phenoxyethanol | 0.70 |
| | Solbrol M | Methylparaben | 0.20 |
| | Solbrol P | Propylparaben | 0.10 |
| | Carbopol ETD 2050 | Carbomer | 0.20 |
| **C** | Natronlauge, 10 % aq. | Sodium Hydroxide | 2.70 |
| **D** | Parfümöl | Parfum (Fragrance) | 0.30 |

### Herstellungsverfahren

- Teil A:: Auf ca. 85°C erhitzen.
- Teil B:: Rohstoffe ohne Carbopol einwiegen. Carbopol mit Ultra Turrax eindispergieren. Auf ca. 85°C erhitzen. B zu A geben.
- Teil C:: Sofort zu A/B geben und anschließend heiß homogenisieren (Ultra Turrax). Unter Rühren abkühlen lassen.
- Teil D:: Zugeben und verrühren.

### Rezepturbeispiel 2

Sonnenschutzlotion (O/W), in-vitro SPF 20

| **Teil** | **Rohstoffe** | **INCI Bezeichnung** | **% (Gew.)** |
|---|---|---|---|
| **A** | Crodafos MCA | Cetyl Phosphate | 1.50 |
| | Cutina MD | Glyceryl Stearate | 2.00 |
| | Copherol 1250 | Tocopherylacetate | 0.50 |
| | Lanette 16 | Cetyl Alcohol | 1.00 |
| | Tegosoft TN | C 12-15 Alkyl Benzoate | 10.60 |
| | Prisorine 3505 | Isostearic Acid | 1.00 |
| | UV-Filter-Verbindung gemäß Formel I | | 2.00 |
| | Neo Heliopan® AV | Ethyl Hexyl Methoxycinnamate | 5.00 |
| **B** | Wasser, dest. | Water (Aqua) | 55.07 |
| | EDETA B fl. | Tetrasodium EDTA | 0.20 |
| | Glycerin, 99% | Glycerin | 3.00 |
| | Phenoxyethanol | Phenoxyethanol | 0.70 |
| | Solbrol M | Methylparaben | 0.20 |
| | Solbrol P | Propylparaben | 0.10 |
| | Carbopol ETD 2050 | Carbomer | 0.20 |
| **C** | Natronlauge, 10 % aq. | Sodium Hydroxide | 3.30 |
| | Neo Heliopan® Hydro, 15% ige Lösung neutralisiert mit NaOH | Phenylbenzimidazole Sulfonic Acid | 13.33 |
| | | | |
| **D** | Parfümöl | Parfum (Fragrance) | 0.30 |

### Herstellungsverfahren

- Teil A:: Auf ca. 85°C erhitzen.
- Teil B:: Rohstoffe ohne Carbopol einwiegen. Carbopol mit Ultra Turrax eindispergieren. Auf ca. 85°C erhitzen. B zu A geben.
- Teil C:: Sofort zu A/B geben und anschließend heiß homogenisieren (Ultra Turrax). Unter Rühren abkühlen lassen.
- Teil D:: Zugeben und verrühren.

### Rezepturbeispiel 3

Sonnenschutzmilch (O/W), in-vitro SPF 6

| **Teil** | **Rohstoffe** | **INCI Bezeichnung** | **% (Gew.)** |
|---|---|---|---|
| **A** | Tegin M | Glyceryl Stearate | 2.50 |
| | Tagat S | PEG-30 Glyceryl Stearate | 1.95 |
| | Lanette O | Cetearyl Alcohol | 2.20 |
| | Copherol 1250 | Tocopherylacetat | 0.50 |
| | Miglyol 8810 | Butylene Glycol Dicaprylate /Caprate | 12.00 |
| | Tegsoft TN | C12 - C15 Alkylbenzoate | 8.00 |
| | Phenonip | Phenoxyethanol (and) Methylparaben (and) Butylparaben (and) Ethyl-paraben (and) Propylparaben | 0.15 |
| | UV-Filter-Verbindung gemäß Formel I | | 5.00 |
| **B** | Wasser, dest. | Water (Aqua) | 43.90 |
| | EDETA BD | Disodium EDETA | 0.10 |
| | 1,2-Propylenglykol | Propylene Glycol | 2.00 |
| | Phenonip | Phenoxyethanol (and) Methylparaben (and) Butylparaben (and) Ethylparaben (and) Propylparaben | 0.30 |
| **C** | Wasser, dest. | Water (Aqua) | 19.00 |
| | Carbopol 2050 | Carbomer | 0.40 |
| | NaOH, 10%ig | Sodium Hydroxide | 1.70 |
| **D** | Parfümöl | Parfum (Fragrance) | 0.30 |

### Herstellungsverfahren

- Teil A:: Auf 80-85°C erhitzen.
- Teil B:: Auf 80-85°C erhitzen, unter Rühren Teil B zu Teil A geben.
- Teil C:: Carbopol in das Wasser eindispergieren und unter Rühren mit NaOH neutralisieren.
Teil C bei ca. 60°C unter Rühren zugeben. Auf RT (Raumtemperatur, 25°C) abkühlen lassen.
- Teil D:: Zugeben und verrühren.

### Rezepturbeispiel 4

Sonnenschutzlotion (O/W), in-vitro SPF 21

| **Teil** | **Rohstoffe** | **INCI Bezeichnung** | **% (Gew.)** |
|---|---|---|---|
| **A** | Tegin M | Glyceryl Stearate | 2.50 |
| | Tagat S | PEG-30 Glyceryl Stearate | 1.95 |
| | Lanette O | Cetearyl Alcohol | 2.20 |
| | Copherol 1250 | Tocopherylacetat | 0.50 |
| | Miglyol 8810 | Butylene Glycol Dicaprylate / Caprate | 12.00 |
| | Tegosoft TN | C12 - C15 Alkylbenzoate | 8.00 |
| | Phenonip | Phenoxyethanol (and) Methylparaben (and) Butylparaben (and) Ethylparaben (and) Propylparaben | 0.20 |
| | UV-Filter-Verbindung gemäß Formel I | | 2.00 |
| | Neo Heliopan® AV | Ethylhexyl Methoxycinnamate | 5.00 |
| | Neo Heliopan® 357 | Butyl Methoxydibenzoylmethane | 1.00 |
| **B** | Wasser, dest. | Water (Aqua) | 39.35 |
| | EDETA BD | Disodium EDETA | 0.10 |
| | 1,2-Propylenglykol | Propylene Glycol | 2.00 |
| | Phenonip | Phenoxyethanol (and) Methylparaben (and) Butylparaben (and) Ethylparaben (and) Propylparaben | 0.30 |
| | Vitamin C | Ascorbic Acid | 0.10 |
| **C** | Wasser, dest. | Water (Aqua) | 20.00 |
| | Carbopol 2050 | Carbomer | 0.40 |
| | NaOH, 10%ig | Sodium Hydroxide | 1.70 |
| **D** | Parfümöl | Parfum (Fragrance) | 0.30 |

### Herstellungsverfahren

- Teil A:: Auf 80-85°C erhitzen.
- Teil B:: Auf 80-85°C erhitzen, unter Rühren Teil B zu Teil A geben.
- Teil C:: Carbopol in das Wasser eindispergieren und unter Rühren mit NaOH neutralisieren.
- Teil C: bei ca. 60°C unter Rühren zugeben. Auf RT abkühlen lassen.
- Teil D:: Zugeben und verrühren.

### Rezepturbeispiel 5

Sonnenschutzlotion (O/W), in-vitro SPF 11

| **Teil** | **Rohstoffe** | **INCI Bezeichnung** | **% (Gew.)** |
|---|---|---|---|
| **A** | Eumulgin VL 75 | Lauryl Glucoside (and) Polyglyceryl-2-Dipolyhydroxystearate (and) Glycerin | 3.00 |
| | Tegosoft TN | C12-25 Alkyl Benzoate | 20.00 |
| | Copherol 1250 | Tocopherylacetat | 0.50 |
| | UV-Filter-Verbindung gemäß Formel I | | 3.00 |
| | Parfümöl | Parfum (Fragrance) | 0.20 |
| | Neo Heliopan® 303 | Octocrylene | 5.00 |
| | Carbopol 2984 | Carbomer | 0.35 |
| | Pemulen TR-1 | Acrylates/C10-30 Alkylacrylate Crosspolymer | 0.15 |
| **B** | Wasser, dest. | Water (Aqua) | 60.50 |
| | EDETA BD | Disodium EDTA | 0.10 |
| | Glycerin, 99% | Glycerin | 5.00 |
| | Phenoxyethanol | Phenoxyethanol | 0.70 |
| | Solbrol M | Methylparaben | 0.20 |
| | Solbrol P | Propylparaben | 0.10 |
| **C** | NaOH, 10%ig | Sodium Hydroxide | 1.20 |

### Herstellungsverfahren

- Teil A:: UV-Absorber gemäß Formel 1 in den Ölen bzw flüssigen UV-Filtern lösen (Erwärmen bis ca. 70°C). Abkühlen lasen auf ca. 30°C, restliche Bestandteile außer Carbopol und Pemulen zufügen und bei Raumtemperatur mischen (ca. 5 Minuten rühren). Carbopol und Pemulen einrühren.
- Teil B:: Solbrole unter Erwärmen in Phenoxyethanol lösen. Mit Wasser und Glycerin mischen, unter Rühren zu Teil A geben. Ca. 60 Minuten rühren.
- Teil C:: Zu A/B geben, mit dem Ultra Turrax homogenisieren.

### Rezepturbeispiel 6

Sonnenschutzcreme (W/O), in-vitro SPF 4. wasserfest

| **Teil** | **Rohstoffe** | **INCI Bezeichnung** | **% (Gew.)** |
|---|---|---|---|
| **A** | Dehymuls PGPH | Polyglyceryl-2 Dipolyhydroxystearate | 5.00 |
| | Copherol 1250 | Tocopherylacetat | 0.50 |
| | Permulgin 3220 | Ozokerite | 0.50 |
| | Zinkstearat | Zinc Stearate | 0.50 |
| | Tegosoft TN | C12-15 Alkyl Benzoate | 25.00 |
| | UV-Filter-Verbindung gemäß Formel I | | 5.00 |
| **B** | Wasser, dest. | Water (Aqua) | 57.90 |
| | EDETA BD | Disodium EDTA | 0.10 |
| | Glycerin, 99% | Glycerin | 4.00 |
| | Phenoxyethanol | Phenoxyethanol | 0.70 |
| | Solbrol M | Methylparaben | 0.20 |
| | Solbrol P | Propylparaben | 0.10 |
| | Magnesiumsulfat | Magnesium Sulfate | 0.50 |

### Herstellungsverfahren

- Teil A:: Auf ca. 85°C erhitzen.
- Teil B:: Auf ca. 85°C erhitzen (ohne Zinkoxid; Zinkoxid mit dem Ultra Turrax eindispergieren).
B zu A geben.
Unter Rühren abkühlen lassen, anschließend homogenisieren.

### Sonnenschutz Softcreme (W/O), in-vitro SPF 40

| **Teil** | **Rohstoffe** | **INCI Bezeichnung** | **% (Gew.)** |
|---|---|---|---|
| **A** | Dehymuls PGPH | Polyglyceryl-2 Dipolyhydroxystearate | 5.00 |
| | Copherol 1250 | Tocopherylacetat | 0.50 |
| | Permulgin 3220 | Ozokerite | 0.50 |
| | Zinkstearat | Zinc Stearate | 0.50 |
| | Tegosoft TN | C12-15 Alkyl Benzoate | 10.00 |
| | UV-Filter-Verbindung gemäß Formel I | | 2.00 |
| | Neo Heliopan® 303 | Octocrylene | 5.00 |
| | Neo Heliopan® MBC | 4-Methylbenzylidene Camphor | 3.00 |
| | Zinkoxid neutral | Zinc Oxide | 5.00 |
| **B** | Wasser, dest. | Water (Aqua) | 62.90 |
| | EDETA BD | Disodium EDTA | 0.10 |
| | Glycerin, 99% | Glycerin | 4.00 |
| | Phenoxyethanol | Phenoxyethanol | 0.70 |
| | Solbrol M | Methylparaben | 0.20 |
| | Solbrol P | Propylparaben | 0.10 |
| | Magnesiumsulfat | Magnesium Sulfate | 0.50 |
| | | | |
| **C** | Parfümöl | Parfum (Fragrance) | 0.20 |

### Herstellungsverfahren

- Teil A:: Auf ca. 85°C erhitzen.
- Teil B:: Auf ca. 85°C erhitzen (ohne Zinkoxid; Zinkoxid mit dem Ultra Turrax eindispergieren).
B zu A geben.
Unter Rühren abkühlen lassen.
- Teil C:: Zugeben und anschließend homogenisieren.

### Rezepturbeispiel 8

Sonnenschutzmilch (W/O)

| **Teil** | **Rohstoffe** | **INCI Bezeichnung** | **% (Gew.)** |
|---|---|---|---|
| **A** | Dehymuls PGPH | Polyglyceryl-2 Dipolyhydroxystearate | 3.00 |
| | Bienenwachs 8100 | Beeswax | 1.00 |
| | Monomuls 90-0-18 | Glyceryl Oleate | 1.00 |
| | Zinkstearate | Zinc stearate | 1.00 |
| | Cetiol SN | Cetearyl Isononanoate | 5.00 |
| | Cetiol OE | Dicaprylyl Ether | 5.00 |
| | Tegosoft TN | C12-15 Alkyl Benzoate | 4.00 |
| | Copherol 1250 | Tocopherylacetat | 0.50 |
| | Solbrol P | Propylparaben | 0.10 |
| | Neo Heliopan® OS | Ethylhexyl Salicylate | 5.00 |
| | Neo Heliopan® AV | Ethylhexyl Methoxycinnamate | 7.50 |
| | UV-Filter-Verbindung gemäß Formel I | | 1.50 |
| **B** | Wasser, dest. | Water (Aqua) | 44.10 |
| | Trilon BD | Disodium EDTA | 0.10 |
| | Glycerin, 99% | Glycerin | 5.00 |
| | Solbrol M | Methylparaben | 0.20 |
| | Phenoxyethanol | Phenoxyethanol | 0.70 |
| | Neo Heliopan® AP 10%ige Lösung neutralisiert mit NaOH | Disodium Phenyl Dibenzimidazole Tetrasulfonate | 15.00 |
| **C** | Parfümöl | Parfum (Fragrance) | 0.30 |
| | Bisabolol | Bisabolol | 0.10 |

### Herstellungsverfahren

- Teil A:: Auf ca. 85°C erhitzen.
- Teil B:: Auf ca. 85°C erhitzen. B zu A geben. Unter Rühren abkühlen lassen.
- Teil C:: Zugeben und anschließend homogenisieren.

### Rezepturbeispiel 9

Tagespflegecreme mit UV-Schutz

| **Teil** | **Rohstoffe** | **INCI Bezeichnung** | **% (Gew.)** |
|---|---|---|---|
| **A** | Emulgade PL 68/50 | Cetearyl Glycoside (and) Cetearyl Alcohol | 4.50 |
| | Cetiol PGL | Hexyldecanol (and) Hexyldecyl Laurate | 8.00 |
| | Myritol 331 | Cocoglycerides | 8.00 |
| | Copherol 1250 | Tocopheryl Acetat | 0.50 |
| | Neo Heliopan® E1000 | Isoamyl-p- Methoxycinnamate | 2.00 |
| | UV-Filter-Verbindung gemäß Formel I | | 2.00 |
| **B** | Wasser, dest | Water (Aqua) | 45.40 |
| | Glycerin | Glycerin | 3.00 |
| | Phenonip | Phenoxyethanol (and) Methylparaben (and) Butyparaben (and) Ethyparaben (and) Propylparaben | 0.50 |
| **C** | Wasser, dest | Water (Aqua) | 25.00 |
| | Carbopol ETD 2050 | Carbomer | 0.20 |
| | NaOH, 10%ig | Sodium Hydroxide | 0.60 |
| **D** | Parfümöl | Parfum (Fragrance) | 0.30 |

### Herstellungsverfahren

- Teil A:: Auf 80°C erhitzen.
- Teil B:: Auf 80°C erhitzen.Unter Rühren zu Teil A geben.
- Teil C:: Carbopol in Wasser dispergieren und mit Natronlauge neutralisieren. Bei ca. 55°C zu Teil A/B geben.
- Teil D:: Bei RT zugeben und homogenisieren.

### Rezepturbeispiel 10

Sonnenschutzspray

| **Teil** | **Rohstoffe** | **INCI Bezeichnung** | **% (Gew.)** |
|---|---|---|---|
| **A** | Wasser, dem. | Water (Aqua) | 69.50 |
| | Glycerin, 99% | Glycerin | 4.00 |
| | 1,3 Butylenglykol | Butylene Glycol | 5.00 |
| | D-Panthenol | Panthenol | 0.50 |
| | Lara Care A-200 | Galactoarabinan | 0.25 |
| **B** | Baysiloneöl M 10 | Dimethicone | 1.00 |
| | Edeta BD | Disodium EDTA | 0.10 |
| | Copherol 1250 | Tocopheryl Acetate | 0.50 |
| | Cetiol OE | Dicaprylyl Ether | 3.00 |
| | Neo Heliopan® HMS | Homosalate | 5.00 |
| | Neo Heliopan® AV | Ethylhexyl Methoxycinnamate | 6.00 |
| | Neo Heliopan® 357 | Butyl Methoxydibenzoylmethane | 1.00 |
| | UV-Filter-Verbindung gemäß Formel I | | 2.00 |
| | alpha Bisabolol nat. | Bisabolol | 0.10 |
| | Pemulen TR-2 | Acrylates/C10-30 Alkyl Acrylate Crosspolymer | 0.25 |
| **C** | Phenoxyethanol | Phenoxyethanol | 0.70 |
| | Solbrol M | Methylparaben | 0.20 |
| | Solbrol P | Propylparaben | 0.10 |
| **D** | NaOH, 10 %ig | Sodium Hydroxide | 0.60 |
| **E** | Parfümöl | Fragrance (Parfum) | 0.20 |

### Herstellungsverfahren

- Teil A:: Lara Care A-200 unter Rühren in den anderen Bestandteilen von Teil A lösen.
- Teil B:: Alle Rohstoffe (ohne Pemulen) einwiegen und die kristallinen Substanzen unter Erwärmen lösen. Pemulen eindispergieren. Teil B zu Teil A geben und 1 Minute homogenisieren.
- Teil C+D: zugeben und nochmals 1-2 Minuten mit dem Ultra Turrax homogenisieren.

### Rezepturbeispiel 11

Sonnenschutz Hydrodispersionsgel (Balm)

| **Teil** | **Rohstoffe** | **INCI Bezeichnung** | **% (Gew.)** |
|---|---|---|---|
| **A** | Wasser, dest. | Water (Aqua) | 74.90 |
| | Carbopol 1342 | Acrylates/C10-30 Alkyl Acrylate Crosspolomer | 1.00 |
| | Triethanolamine | Triethanolamine | 1.20 |
| **B** | Neo Heliopan® Hydro, 30%ige Lösung neutralisiert mit TEA | Phenylbenzimidazole Sulfonic Acid | 10.00 |
| **C** | Neo Heliopan® AV | Ethylhexyl Methoxycinnamate | 3.00 |
| | UV-Filter-Verbindung gemäß Formel I | | 2.00 |
| | Isopropylmyristat | Isopropyl Myristate | 4.00 |
| | Baysilone ÖL PK 20 | Phenyl Trimethicone | 3.00 |
| | Phenonip | Phenoxyethanol (and) Methylparaben (and) Butyparaben (and) Ethyparaben (and) Propylparaben | 0.50 |
| | Parfümöl | Parfum (Fragrance) | 0.30 |
| | Bisabolol nat | Bisabolol | 0.10 |

### Herstellungsverfahren

- Teil A:: Carbopol in Wasser dispergieren und mit Natronlauge neutralisieren.
- Teil B:: Unter Rühren zu Teil A geben.
- Teil C:: Kristalline Bestandteile unter Erwärmen (max.40°C) in den anderen Rohstoffen von Teil C lösen und zu Teil A/B geben. Gut verrühren und anschließend homogenisieren. (Homozenta).

### Rezepturbeispiel 12

Hair Conditioner mit UV-Filtern

| **Teil** | **Rohstoffe** | **INCI Bezeichnung** | **% (Gew.)** |
|---|---|---|---|
| **A** | Emulgade 1000 NI | Cetearyl Alcohol (and) Ceteareth-20 | 2.00 |
| | Lanette 16 | Cetyl Alcohol | 1.00 |
| | Neo Heliopan® AV | 2 -Ethylhexyl Methoxycinnamate | 3.00 |
| | UV-Filter-Verbindung gemäß Formel I | | 1.00 |
| **B** | Wasser, dest | Water (Aqua) | 91.70 |
| | Edeta BD | Disodium EDTA | 0.10 |
| | Phenonip | Phenoxyethanol (and) Methylparaben (and) Butyparaben (and) Ethyparaben (and) Propylparaben | 0.40 |
| | Dehyquart A-CA | Cetrimonium Chloride | 0.20 |
| | NaOH, 1%ig | Sodium Hydroxide | 0.30 |
| **C** | Parfümöl | Parfum ( Fragrance) | 0.30 |

### Herstellungsverfahren

- Teil A:: Auf 80°C erhitzen.
- Teil B:: Auf 80°C erhitzen. Unter Rühren zu Teil A geben.
- Teil C:: Bei 40°C zugeben und auf RT abkühlen.

### Rezepturbeispiel 13

Sonnenschutzlotion (O/W)

| **Teil** | **Rohstoffe** | **INCI Bezeichnung** | **% (Gew.)** |
|---|---|---|---|
| **A** | Tegin M | Glyceryl Stearate | 2.50 |
| | Tagat S | PEG-30 Glyceryl Stearate | 1.95 |
| | Lanette O | Cetearyl Alcohol | 2.20 |
| | Hallbrite TQ | Diethylhexylnaphthalate | 7,00 |
| | Cetiol B | Dibutyl Adipate | 5,00 |
| | Tegosoft TN | C12 - C15 Alkylbenzoate | 4.00 |
| | Myritol PC | Propylene Glycol Dicaprylate/Dicaprate | 4.00 |
| | Phenonip | Phenoxyethanol (and) Methylparaben (and) Butylparaben (and) Ethyl-paraben (and) Propylparaben | 0.15 |
| | UV-Filter-Verbindung gemäß Formel I | | 2.00 |
| | Neo Heliopan® AV | Ethylhexyl Methoxycinnamate | 5.00 |
| **B** | Wasser, dest. | Water (Aqua) | 42,80 |
| | 1,2-Propylenglykol | Propylene Glycol | 2.00 |
| | Phenonip | Phenoxyethanol (and) Methylparaben (and) Butylparaben (and) Ethylparaben (and) Propylparaben | 0.30 |
| **C** | Wasser, dest. | Water (Aqua) | 19.00 |
| | Carbopol 2050 | Carbomer | 0.40 |
| | NaOH, 10%ig | Sodium Hydroxide | 1.70 |

### Herstellungsverfahren

- Teil A:: Auf 80-85°C erhitzen.
- Teil B:: Auf 80-85°C erhitzen, unter Rühren Teil B zu Teil A geben.
- Teil C:: Carbopol in das Wasser eindispergieren und unter Rühren mit NaOH neutralisieren. Teil C bei ca. 60°C unter Rühren zugeben.

### Beispiele zur Photostabilitätsprüfung:

Mit einem Suntester der Fa. Heräus wurden Photostabilitätsprüfungen durchgeführt. Die Bestrahlungsstärke betrug dabei 80 W/m², bezogen auf den UV-Bereich von 290-400 nm. Die Bestrahlungszeit betrug insgesamt 4h, wobei der Photoab-bau der UV-Filter nach 2 und 4h Bestrahlungsdauer anhand von HPLC-Analysen gemessen wurde. Die Bestrahlungen der UV-Filter-Mischungen wurden in einer Isopropylmyristat-Lösung durchgeführt. Die prozentualen Werte beziehen sich auf den gemessenen Wert ohne Bestrahlung (Abbau der Konzentration).

### Vergleichsmessung (Bezug)

3% Octylmethoxycinnamat (OMC)
1% 4-Dimethylethyl-4'-methoxydibenzoylmethan (DMDBM)

| **Zeit** | **OMC** | ***DMDBM*** |
|---|---|---|
| **2h** | 58% | 67% |
| **4h** | 76% | 87% |

Nachfolgend werden erfindungsgemäß zu verwendende Verbindungen A1, A2 und nicht erfindungsgemäß zu verwendende Verbindungen B1, B2 und B3 verglichen.

### Versuch 1 (erfindungsgemäß):

3% Octylmethoxycinnamat (OMC)
1% 4-Dimethylethyl-4'-methoxydibenzoylmethan (DMDBM)
2% Verbindung **A1** (n-Butylester), bevorzugte Verbindung der Formel I mit
- R¹: Methoxy, das in para-Position zu dem die Substituenten R₄, R₅ und R₆ tragenden Rest angeordnet ist,
- R² und R³: Wasserstoff,
- R⁴: CO₂R mit R gleich n-Butyl
- R⁵: H und
- R⁶: Phenyl

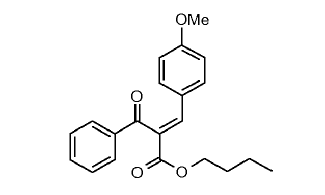

### A1

| ***Zeit*** | ***A1*** | ***OMC*** | ***DMDBM*** |
|---|---|---|---|
| **2h** | 0% | 12% | 24% |
| **4h** | 0% | 17% | 38% |

Die Untersuchung ergab eine hervorragende Photostabilität nicht nur der Verbindung A1 selbst, sondern auch der Co-UV-Filter OMC und DMDBM im Vergleich mit der Vergleichsmessung.

Anmerkung: Weitere, hier nicht im Einzelnen wiedergegebene Versuche ergaben für sämtliche Verbindungen (A₁-Alkylester) mit 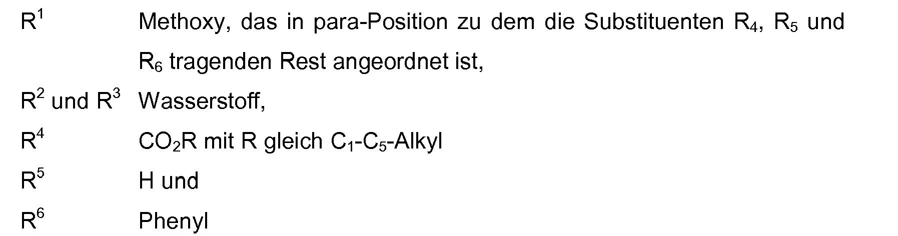 ähnlich gute Photostabilitätswerte.

### Versuch 2 (erfindungsgemäß)

3% Octylmethoxycinnamat (OMC)
1% 4-Dimethylethyl-4'-methoxydibenzoylmethan (DMDBM)
2% Verbindung **A2 (Anmerkung: A2 ist ein Feststoff)**

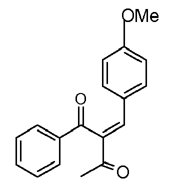

| ***Zeit*** | ***A2*** | ***OMC*** | ***DMDBM*** |
|---|---|---|---|
| **2h** | 3% | 11% | 20% |
| **4h** | 3% | 17% | 28% |

### Versuch 3 (erfindungsgemäß)

3% Octylmethoxycinnamat (OMC)
1% 4-Dimethylethyl-4'-methoxydibenzoylmethan (DMDBM)
2% Verbindung **A3**

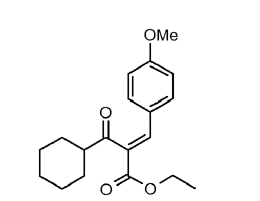

| ***Zeit*** | ***A2*** | ***OMC*** | ***DMDBM*** |
|---|---|---|---|
| **2h** | 0% | 11% | 20% |
| **4h** | 0% | 17% | 28% |

### Versuch 4 (nicht erfindungsgemäß)

3% Octylmethoxycinnamat (OMC)
1% 4-Dimethylethyl-4'-methoxydibenzoylmethan (DMDBM)
2% Verbindung **B1**

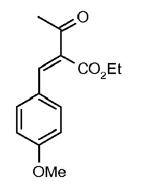

| ***Zeit*** | ***B1*** | ***OMC*** | ***DMDBM*** |
|---|---|---|---|
| **2h** | 7% | 11% | 27% |
| **4h** | 12% | 18% | 48% |

### Versuch 5 (nicht erfindungsgemäß):

3% Octylmethoxycinnamat (OMC)
1% 4-Dimethylethyl-4'-methoxydibenzoylmethan (DMDBM)
2% Verbindung **B2**

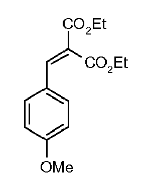

| ***Zeit*** | ***B2*** | ***OMC*** | ***DMDBM*** |
|---|---|---|---|
| **2h** | 2% | 15% | 27% |
| **4h** | 7% | 30% | 60% |

### Versuch 6 (nicht erfindungsgemäß):

3% Octylmethoxycinnamat (OMC)
1% 4-Dimethylethyl-4'-methoxydibenzoylmethan (DMDBM)
2% Verbindung **B3**

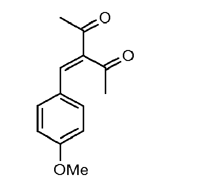

| ***zeit*** | ***B3*** | ***OMC*** | ***DMDBM*** |
|---|---|---|---|
| **2h** | 0% | 13% | 27% |
| **4h** | 8% | 25% | 52% |

### Beispiele zur Löslichkeit (in Gewichtsprozent)

| **Substanz** | **Isopropylmyristat** | **Miglyol-812** | **Witconol-TN** |
|---|---|---|---|
| A1 - Methylester | <10% | <10% | <10% |
| A1 - Ethylester | <10% | <10% | <10% |
| A1 - Isopropylester | >10% | >10% | >10% |
| A1 - n-Propylester | >10% | >10% | >10% |
| A1 - Isobutylester | >20% | >20% | >20% |
| A1 - n-Butylester | >20% | >20% | >20% |
| A1 - Isoamylester | >20% | >20% | >20% |
| A2 | <10% | <10% | <10% |
| A3 | >20% | >20% | >20% |

(Angaben in Gewichtsprozent)
Zu den Strukturen der untersuchten Verbindungen vergleiche die Versuche 1 - 3.

## Patentansprüche

1. Verwendung einer Verbindung der Formel 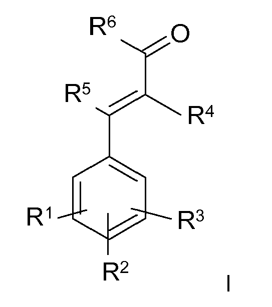 worin
R¹-R³ unabhängig voneinander Wasserstoff, C₁-C₈-Alkyl oder C₁-C₈-Alkoxy,
R⁴ COR, CO₂R, CONR₂ mit R gleich C₁-C₈-Alkyl oder C₃-C₈-Cycloalkyl
R⁵ H oder C₁-C₈-Alkyl,
R⁶ Aryl, mit bis zu drei C₁-C₈-Alkyl- oder C₁-C₈-Alkoxy substituiertes
Aryl, oder C₃-C₈-Cycloalkyl
sind,
als UV-Filter in kosmetischen Formulierungen, insbesondere in Kombination mit UV-Filtern aus der Gruppe der Methoxycinnamat-Derivate und/oder Dibenzoylmethan-Derivate, mit der Maßgabe, dass R⁵ gleich H ist, wenn R⁴ gleich COR ist.

2. Verwendung nach Anspruch 1, wobei
R¹-R³ unabhängig voneinander Wasserstoff, C₁-C₈-Alkyl oder C₁-C₈-Alkoxy,
R⁴ CO₂R mit R gleich C₁-C₈-Alkyl,
R⁵ H oder C₁-C₈-Alkyl,
R⁶ Aryl oder mit bis zu drei C₁-C₈-Alkyl- oder C₁-C₈-Alkoxy substituiertes
Aryl
sind.

3. Verwendung nach Anspruch 1 oder 2, wobei
R¹ Methoxy,
R² und R³ H,
R⁴ CO₂R mit R gleich C₂-C₅-Alkyl und
R⁶ Phenyl oder mit bis zu drei C₁-C₈-Alkyl- oder C₁-C₈-Alkoxy substituiertes Phenyl
sind.

4. Verwendung nach einem der Ansprüche 1-3, wobei
R¹ Methoxy, das in para-Position zu dem die Substituenten R₄, R₅ und
R₆ tragenden Rest angeordnet ist,
R² und R³ H,
R⁴ CO₂R mit R gleich C₄-C₅-Alkyl,
R⁵ H oder C₁-C₈-Alkyl, und
R⁶ Phenyl
sind.

5. Verwendung nach Anspruch 1, wobei
R⁶ C₃-C₈-Cycloalkyl ist.

6. Verwendung nach Anspruch 5, wobei
R⁶ Cyclohexyl ist.

7. Verwendung nach Anspruch 5 oder 6, wobei
R⁴ CO₂R mit R gleich C₁-C₈-Alkyl ist.

8. Verwendung nach einem der Ansprüche 5, 6 oder 7, wobei
R⁴ CO₂R mit R gleich Ethyl ist.

9. Kosmetische oder dermatologische Formulierung umfassend
- eine oder mehrere Verbindungen der Formel I wie in einem der vorangehenden Ansprüche definiert, als UV-Absorber.

10. Kosmetische oder dermatologische Formulierung nach Anspruch 9, weiter umfassend:
- einen oder mehrere weitere UV-Absorber, insbesondere aus der Gruppe der Methoxycinnamat-Derivate und/oder Dibenzoylmethan-Derivate
und/oder
- umhüllte oder nicht umhüllte Pigmente von Metalloxiden.

11. Kosmetische oder dermatologische Formulierung nach Anspruch 10, wobei die eingesetzten UV-Absorber bzw. Pigmente so ausgewählt und in ihrer jeweiligen Menge so aufeinander abgestimmt sind, dass sie derart zusammenwirken, dass der Sonnenschutzfaktor der Formulierung synergistisch erhöht ist.

12. Kosmetische oder dermatologische Formulierung nach Anspruch 10, wobei die eingesetzten UV-Absorber so ausgewählt und in ihrer jeweiligen Menge so aufeinander abgestimmt sind, dass die kritische Wellenlänge der Formulierung λ_{crit.}>380 nm ist.

13. Verbindung der Formel 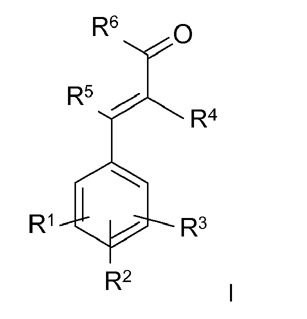 worin
R¹ C₁-C₈-Alkoxy das in para-Position zu dem die Substituenten R⁴, R⁵ und R⁶ tragenden Rest angeordnet ist,
R² und R³ Wasserstoff,
R⁴ CO₂R mit R gleich C₁-C₈-Alkyl,
R⁵ H,
R⁶ Phenyl oder Cyclohexyl
ist.

14. Verbindung nach Anspruch 13, wobei
R⁴ CO₂R mit R gleich Methyl, Ethyl, n-Propyl, iso-Propyl, n-Butyl, sec-Butyl, iso-Butyl, tert-Butyl oder iso-Amyl und
R⁶ Phenyl
ist.

15. Verbindung nach Anspruch 14, wobei
R⁴ CO₂R mit R gleich Methyl, Ethyl, n-Propyl, iso-Propyl, n-Butyl, sec-Butyl, iso-Butyl oder tert-Butyl und
R⁶ Cyclohexyl
ist.
